# EUROPÄISCHE PATENTANMELDUNG

(11) **EP 2 163 205 A1**
(43) Veröffentlichungstag der Anmeldung: **17.03.2010**
(21) Anmeldenummer: 08015988.2
(22) Anmeldetag: 11.09.2008
(51) Int. Cl.: A61B 17/00, A61F 13/00, A61J 15/00, A61M 1/00

(54) **Vorrichtung zum Verschliessen von Wunden, insbesondere von Wunden, die durch einen Eingriff mittels eines Trokars in der Bauchdecke entstehen**

(71) Anmelder: Wicky, Beat, Dr., 6300 Zug (CH)
(72) Erfinder: Wicky, Beat, Dr., 6300 Zug (CH)
(74) Vertreter: Schmauder & Partner AG Patent- & Markenanwälte VSP

(57) **Zusammenfassung**

Um eine Vorrichtung zum Verschliessen von Wunden, die durch einen Eingriff mittels eines Trokars (20), insbesondere in der Bauchdecke, entstehen, zu verbessern und insbesondere einerseits verschlusssicher und andererseits verrutschsicher zu machen, wird vorgeschlagen, eine solche Vorrichtung mit einem scheibenförmigen Teil (4), vorzugsweise zur innenseitigen Anlage an das Peritoneum (30), einem vorzugsweise stabförmigen Anschlussteil (6), der sich proximal an den scheibenförmigen Teil anschliesst und mit diesem verbunden ist, und einem Verbindungselement zum lösbaren Verbinden der Vorrichtung mit einem Applizierelement (22) zu versehen und dadurch zu verbessern, dass am proximalen Ende des Anschlussteils (6) eine Verankerungseinrichtung (8) angeordnet ist, mit der die Vorrichtung im Gewebe mittels Widerhaken (12) verankerbar ist.

## Beschreibung

### Technisches Gebiet

Die Erfindung betrifft eine Vorrichtung zum Verschliessen von Wunden. Insbesondere betrifft die Erfindung eine Vorrichtung zum Verschliessen von Wunden, die durch das Einführen von Trokaren durch die Bauchdecke entstehen, sowie Organperforationen im Rahmen der transluminalen endoskopischen Eingriffe durch natürliche. Körperöffnungen ("natural orifice transluminal endoscopic surgery", "NOTES").

### Stand der Technik

Bei der Laparoskopie werden durch Trokare unterschiedlichen Durchmessers optische Systeme und Instrumente in den Körper eingeführt. So werden grössere Öffnungen im Bereich der Bauchdecke vermieden. Die Laparoskopie stellt ein Verfahren dar, bei dem der Bauchraum und die sich darin befindlichen Organe wie Leber, Milz, Magen, Dünndarm, Dickdarm, gynäkologische Organe usw. mit einem sogenannten Endoskop gespiegelt werden. Kaum eine Entwicklung hat die moderne Medizin derart beeinflusst wie die Innovationen im Bereiche der Endoskopie. Dabei ist zu unterscheiden zwischen der diagnostischen Laparoskopie und der therapeutischen Laparoskopie. Bei der diagnostischen Laparoskopie geht es um die Stellung einer Diagnose durch Betrachtung der Organe innerhalb der Bauchhöhle und allenfalls durch die Entnahme einer Gewebeprobe für die histologische ("feingewebliche") Untersuchung. Die therapeutische Laparoskopie ermöglicht die Behandlung von krankhaften Veränderungen im Bauchraum auf endoskopischem Weg. Dabei kommen sogenannt "minimal invasive Operationsverfahren" zur Anwendung, die für den Patienten eine sehr schonende Alternative zu einer offenen Operation darstellen. Es können heute eine grosse Anzahl von chirurgischen Eingriffen im Rahmen einer therapeutischen Laparoskopie durchgeführt werden. Das gilt insbesondere für die Appendektomie (Blinddarmoperation), Cholezystektomie (Gallenblasenentfernung), Herniotomie (Leistenbruchoperation), Dickdarmresektion, Adhäsiolyse (Lösung von Verwachsungen) und eine Vielzahl von gynäkologischen Eingriffen.

Bei der erst in der Anfangsphase der Entwicklung stehenden NOTES werden optische Systeme und Instrumente durch natürliche Körperöffnungen (durch den Magen, durch die Vagina, durch das Rektum usw.) eingeführt. Durch Perforation der entsprechenden Organe kann von dort aus die freie Bauchhöhle zwecks diagnostischer und therapeutischer Massnahmen erreicht werden. Nach Rückzug der Instrumente müssen diese Öffnungen ebenfalls korrekt verschlossen werden.

Das laparoskopische Operieren ist chirurgisch anspruchsvoll und erfordert eine moderne technische Infrastruktur. Dazu gehören ein modernes Bildübertragungssystem, zuverlässige Apparaturen und elektronische Kontrollsysteme sowie spezielle Operationsinstrumente, die durch die Medizintechnik in den letzten Jahren in zunehmender Verfeinerung entwickelt wurden.

Um die Wunden eines laparoskopischen Eingriffs zu verschliessen, kommen zunächst klassische Verfahren in Betracht, insbesondere das chirurgusche Vernähen der unter der Bauchhaut liegenden Faszie, also der tragenden Faserschicht der Bauchdecke. Dies kann sich als technisch schwierig und zeitraubend erweisen. Wird eine Trokarwunde ab einer Trokargrösse ab 1 cm nicht korrekt verschlossen, drohen gefährliche Komplikationen als Folge einer Trokarhernie. Auf Grund der bestehenden Literaturangaben wird die Inzidenz einer Trokarhernie heute auf 1-2% geschätzt. Von einer Trokarhernie wird gesprochen, wenn Bauchorgane in einer nicht verschlossenen Bauchwandlücke eingeklemmt werden. Handelt es sich dabei um Anteile des Darms, kann sich ein potenziell lebensbedrohlicher Darmverschluss(lleus) einstellen, der in der Regel eine notfallmässige Zweitoperation erfordert. Um diese dramatische Entwicklung zu vermeiden, wird generell empfohlen, Trokareinstichstellen ≥ 1 cm adäquat zu verschliessen. Dabei wurden diverse Verschlussvorrichtungen vorgeschlagen.

Zur weiteren Erörterung wird zunächst einmal die Nomenklatur erläutert, die im nachfolgenden benutzt wird. Die Begriffe "distal" und "proximal" werden für die Instrumente vom Operateur aus gesehen benutzt. "Distal" soll also der Teil oder das Ende bedeuten, welches von der Hand des Operateurs am weitesten entfernt ist, "proximal" der Teil oder das Ende, welches der Hand der Operateurs nächstliegend ist.

Aus der US 5 478 354 ist eine Verschlussvorrichtung bekannt (Fig. 39 - 41), bei der eine Unterlegscheibe durch ein clipartiges Element, welche das distale Ende der Verschlussvorrichtung darstellt, an der Fascie gehalten wird. Die Vorrichtung wird durch den Operationstrokar mittels eines Applizierstabes eingeführt. Mit dem Applizierstab wird das clipartige Element, welches Beinchen mit Widerhaken aufweist, in die Fascie hereingezogen. Gemäss der Erfindung der US 5 478 354 ist das clipartige Element so ausgebildet, dass bei einem Zusammenwirken mit der Unterlegscheibe die Beinchen nach innen zum Applizierstab gedrückt werden und sich dort verankern sollen. Der Applizierstab hat ein Gewinde und soll dann aus dem clipartigen Element herausgeschraubt und aus der Laparoskopieöffnung entfernt werden. Dieses Konzept hat sich nicht als besonders vorteilhaft herausgestellt, da insbesondere die Möglichkeit besteht, dass sich die Vorrichtung aus dem Gewebe löst und in den Bauchraum wandert. Auch andere schirmartig ausgebildete Vorrichtungen, bei denen eine Art Schirm nach aussen in die Fascie bzw. in das Peritoneum aufgeklappt werden, haben sich - aus ähnlichen Gründen - als nur beschränkt tauglich erwiesen.

### Darstellung der Erfindung

Aufgabe der Erfindung ist es, eine Vorrichtung vorzuschlagen, mit der eine Trokaröffnung sicher verschlossen werden kann, ohne dass es zu einer Trokarhernie kommen kann oder dass die Vorrichtung sich aus dem Gewebe löst.

Die Aufgabe der Erfindung wird durch eine Vorrichtung nach Anspruch 1 gelöst. Dabei haben die Massnahmen der Erfindung zunächst einmal zur Folge, dass bei einer Vorrichtung zum Verschliessen von Wunden, die durch einen Eingriff mittels eines Trokars entstehen, wobei die Vorrichtung einen scheibenförmigen Teil, einen Anschlussteil, der sich proximal an den scheibenförmigen Teil anschliesst und mit diesem verbunden ist, und ein Verbindungselement zum lösbaren Verbinden der Vorrichtung mit einem Manipulierelement aufweist, die Verankerbarkeit dadurch verbessert wird, dass am proximalen Ende des Anschlussteils eine Verankerungseinrichtung angeordnet ist, mit der die Vorrichtung im Gewebe, mittels Widerhaken verankerbar ist. Es sollte in diesem Zusammenhang bemerkt werden, dass - je nach Anforderungen - der scheibenförmige Teil nicht rund sein muss, sondern auch als scheibenförmiges Vieleck oder im weitesten Sinn oval ausgebildet sein kann. Weiterhin wird der scheibenförmige Teil zumeist in seiner Ausdehnung so ausgebildet sein, dass er kleiner ist als der Innendurchmesser des Trokars, für den die Vorrichtung vorgesehen ist. Es ist aber auch möglich, den scheibenförmigen Teil elastisch oder biegbar mit einer grösseren Ausdehnung auszubilden, so dass er in eingeklapptem oder gestauchtem Zustand durch den Trokar hindurchbewegt wird und sich erst danach voll entfaltet.

Besonders vorteilhaft ist die Ausführung der Erfindung, bei der die Verankerungseinrichtung schirmartig ausgebildet ist und eine Vielzahl von Widerhaken aufweist (Anspruch 2). Dabei ist es vorteilhaft, wenn die Verankerungseinrichtung aufklappbar ist, da dann z.B. eine Verankerungsfläche ermöglicht wird, die grösser ist als der Durchmesser des Trokars (Anspruch 3). Dieser Effekt kann vorteilhafter Weise dadurch erreicht werden, dass die Verankerungseinrichtung dadurch aufklappbar ist, dass die Widerhaken jeweils um eine Achse senkrecht zum Anschlussteil schwenkbar oder biegbar sind (Anspruch 4).

Die Vorrichtung der Erfindung kann einstückig ausgebildet sein (Anspruch 5), indem z.B. der scheibenförmige Teil, der Anschlussteil und die Verankerungseinrichtung aus einem Stück gegossen sind. Besonders vorteilhaft ist es aber, wenn die Vorrichtung der Erfindung zweiteilig aufgebaut ist (Anspruch 6). Dabei kann der Anschlussteil, vorzugsweise an seinem proximalen Ende, im Wesentlichen stabförmig ausgebildet sein, und die Verankerungseinrichtung einen ringförmigen, um den Anschlussteil herum angeordneten Mittelteil aufweisen (Anspruch 7). Der Mittelteil der Verankerungseinrichtung kann vorteilhafter Weise längs des Anschlussteils verschiebbar und vorzugsweise an dessen proximalen Ende durch eine Einrastung arretierbar sein, wodurch der Aufklappmechanismus besonders vorteilhaft ausgebildet ist (Anspruch 8 und 9).

Vorteilhaft ist es, wenn am scheibenförmigen Teil ebenfalls Verankerungselemente angeordnet sind, die im Wesentlichen parallel zum Anschlussteil ausgerichtet sein können. Wenn diese weiteren Verankerungselemente an ihrem Ende Widerhaken aufweisen, so sind eine sehr gute Positionierung der Vorrichtung und damit ein optimaler Wundverschluss möglich (Anspruch 10).

Nicht Gegenstand der Erfindung selbst ist ein Manipulierelement, mit dem die Vorrichtung der Erfindung, vorzugsweise durch den Trokar, in den Körper eingeführt und an der Verschlussstelle positioniert wird. Dieses beispielsweise als Stab ausgebildete Applizier- bzw. Manipulierelement wird nach dem Wundverschluss entfernt. Dazu wird es mit einem Mechanismus ausgebildet sein, der mit dem Anschlussteil der Vorrichtung der Erfindung zusammenwirkt. Eine einfache Einrichtung ist dabei, wenn der Anschlussteil ein Gewinde aufweist, in oder auf welches ein Applizierelement mit einem entsprechenden Gegengewinde schraubbar und aus diesem heraus schraubbar ist (Anspruch 11).

Der Anschlussteil kann aber auch eine oder mehrere Ausnehmungen aufweisen, in die eine entsprechende, lösbare Einrichtung des Applizier- bzw. Manipulierelements eingreifen kann (Anspruch 12). Besonders vorteilhaft ist es, wenn der Anschlussteil einen Schnellverschluss zum Verbinden und Lösen des Manipulierelements aufweist (Anspruch 13).

Vorteilhaft ist es, wenn die Vorrichtung der Erfindung zumindest teilweise, vorzugsweise aber vollständig, aus bioabbaubarem Material gebildet ist. Dabei kommen insbesondere Lactidpolymere, aber auch andere im Körper leicht abbaubare Materialien in Frage (Anspruch 14).

Eine besonders vorteilhafte Ausgestaltung der Erfindung kann dadurch erreicht werden, wenn der Anschlussteil röhrenartig ausgebildet ist, und an der Vorrichtung am proximalen und am distalen Ende Anschlusseinrichtungen zum Durchführen von Ver- und/oder Entsorgungselementen - z.B. Blasenkatheter, Zuführungen für eine künstliche Ernährung etc. aufweist. Der scheibenförmige Teil wird dabei einen lochartigen Durchlass als Verlängerung der Röhre aufweisen (Anspruch 15).

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können.

Es sollte beachtet werden, dass die hier beschriebene Vorrichtung nicht nur zum Verschliessen von Trokarwunden geeignet ist, die durch laparoskopische Eingriffe durch die Bauchdecke, also durch Faszie und Peritoneum hindurch entstehen, sondern auch für solche, die durch endoskopische Operationen durch natürliche Körperöffnungen (NOTES) hindurch geführt werden.

### Kurze Beschreibung der Zeichnungen

Weitere Einzelheiten, Vorteile und Merkmale des Gegenstandes der vorliegenden Erfindung ergeben sich aus der nachfolgenden Beschreibung der dazu gehörenden Zeichnungen, in denen - beispielhaft - erfindungsgemässe Vorrichtungen erläutert werden. In den Zeichnungen zeigt:
- Figur 1: eine perspektivische Darstellung der Vorrichtung mit einem Appli-zierstab in der Vorbereitungsphase der Anwendung, gemäss einem ersten, zweiteilig ausgebildeten Ausführungsbeispiel der vorliegen-den Erfindung;
- Figur 2: eine perspektivische Darstellung der Vorrichtung nach Figur 1 nach der Anwendung und daher ohne den Applizierstab;
- Figur 3: eine Darstellung gemäss Figur 2, in Ansicht von oben, also auf die Wunde;
- Figur 4: eine Ansicht der Vorrichtung gemäss Figur 1 bis 3 von der Seite mit schematischer Darstellung des Appliziervorganges;
- Figur 5: eine Ansicht der Vorrichtung gemäss Figur 1 bis 3 von der Seite nach Ende des Appliziervorganges;
- Figur 6: eine Darstellung der Vorrichtung mit einem Applizierstab in der Vorbereitungsphase der Anwendung, gemäss einem zweiten, ein-teilig ausgebildeten Ausführungsbeispiel der vorliegenden Erfin-dung;
- Figur 7: eine Darstellung der Vorrichtung mit einem Applizierstab in der Ap-plizierphase der Anwendung, gemäss dem zweiten Ausführungs-beispiel nach Figur 6;
- Figur 8: eine Darstellung der Vorrichtung mit einem Applizierstab nach der Applizierphase der Anwendung, gemäss dem zweiten Ausfüh-rungsbeispiel nach Figur 6 und 7;
- Figur 9: eine perspektivische Darstellung der Vorrichtung, gemäss einer alternativen Ausführung der vorliegenden Erfindung, mit Beinchen und Widerhaken an der Scheibe.

### Wege zur Ausführung der Erfindung

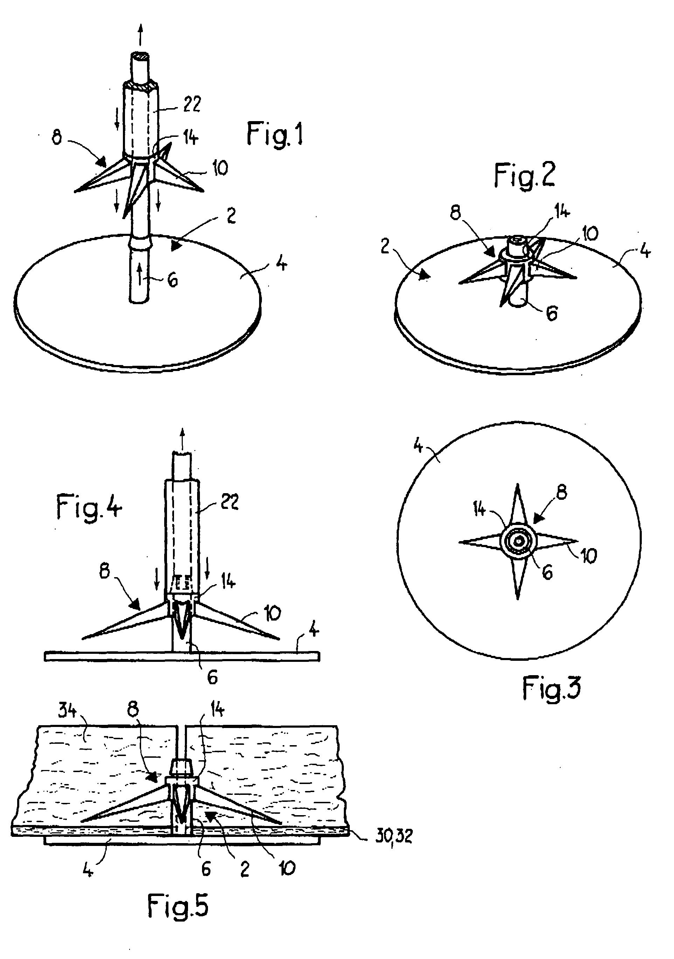

Die in den Figuren 1 bis 5 dargestellte Vorrichtung 2 zum Verschliessen von Wunden, die durch einen Eingriff mittels eines Trokars 20 entstehen, weist zunächst einmal einen scheibenförmigen Teil 4, einen Anschlussteil 6, der sich proximal an den scheibenförmigen Teil 4 anschliesst und mit diesem verbunden ist, und ein Verbindungselement zum lösbaren Verbinden der Vorrichtung mit einem Applizier- bzw. Manipulierelement 22 auf. Am proximalen Ende des Anschlussteils 6 ist eine Verankerungseinrichtung 8 angeordnet, mit der die Vorrichtung 2 im Gewebe 30, 32, 34 mittels Verankerungselemente 10, die hier als Widerhaken ausgebildet sind, verankerbar ist. Die Verankerungseinrichtung 8 ist schirmartig ausgebildet und weist eine Vielzahl von Verankerungselementen - in der Figur 4 Widerhaken - auf. Die Verankerungseinrichtung ist aufklappbar, wodurch eine grosse Verankerungsfläche ermöglicht wird, die grösser ist als der Durchmesser des Trokars 20. Im hier beschriebenen Ausführungsbeispiel sind dabei die Widerhaken jeweils um eine Achse senkrecht zum Anschlussteil schwenkbar oder biegbar. In diesem Ausführungsbeispiel ist die Vorrichtung der Erfindung zweiteilig aufgebaut. Dabei ist der Anschlussteil 6 an seinem proximalen Ende im Wesentlichen stabförmig ausgebildet, und die Verankerungseinrichtung 8 weist einen ringförmigen, um den Anschlussteil 6 herum angeordneten Mittelteil 14 auf. Der Mittelteil 14 ist bezüglich des Anschlussteils 6 verschiebbar und an dessen proximalen Ende durch eine auskragende Wulst arretierbar.

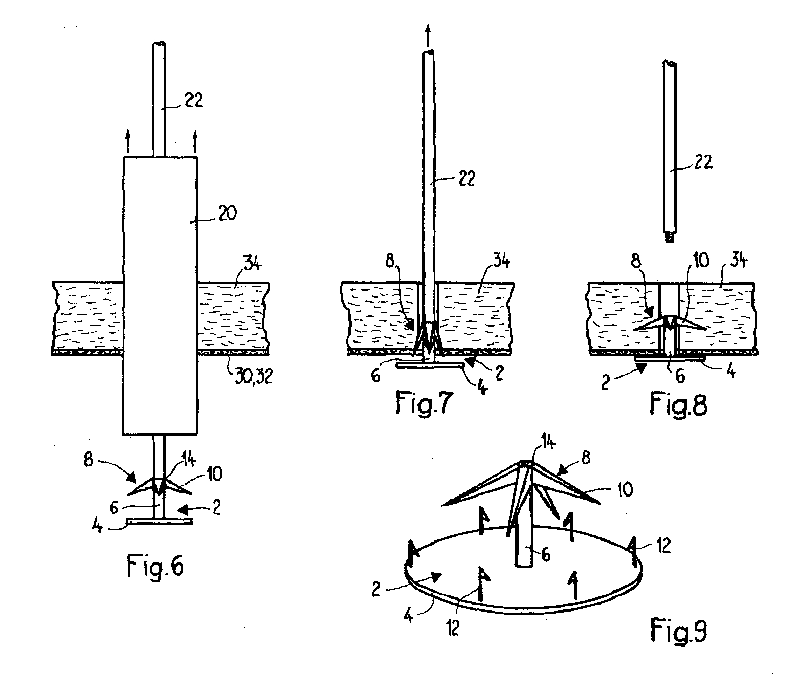

In einem zweiten Ausführungsbeispiel der vorliegenden Erfindung gemäss den Figuren 6 bis 8 ist die Vorrichtung der Erfindung einstückig ausgebildet, indem der scheibenförmige Teil 4, der Anschlussteil 6 und die Verankerungseinrichtung 8 aus einem Stück gegossen sind. Der Aufklappmechnismus wird hier durch eine Vorspannung bewirkt, die sich im Gewebe 30, 32, 34 löst. Es sollte in diesem Zusammenhang bemerkt werden, dass die Dickeverhältnisse zwischen Fettgewebe 34, Peritoneum 32 und Fascie 30 mit Muskel sowohl in Hinblick auf den Patienten wie auch in Hinblick auf die Operationsstelle sehr unterschiedlich sind und daher in diesem Zusammenhang schematisch aufzufassen sind.

In dieser Ausführung ist ein Applizierstab 22 mit der Vorrichtung 2 verbunden und wird durch den Trokar in einen - üblicherweise durch Einleiten eines Gases (CO₂) -künstlich erzeugten Hohlraum im Bauchraum eingeführt (Figur 6) und an der Verschlussstelle positioniert. Dabei wird der Trokar 20 herausgezogen und hinterlässt eine wesentlich kleinere Wundöffnung als der Durchmesser des Trokars 20. Die Vorrichtung 2 wird nun mittels des Applizierstabs 22 von innen in die Wundöffnung gezogen, wodurch die Verankerungseinrichtung 8 mit den als biegbare Widerhaken ausgebildeten Verankerungselementen 10 zusammengezogen wird. In diesem Ausführungsbeispiel wird der Applizierstab 22 nun herausgeschraubt oder - bei einer alternativen Ausgestaltung der Vorrichtung - aus den dazu vorgesehenen Ausnehmungen im proximalen Teil der Vorrichtung oder durch Öffnung eines bajonettartigen Schnellverschlusses gelöst.

In einer alternativen Ausführung, die auf alle vorstehend beschriebenen Ausführungsformen anwendbar ist, sind am scheibenförmigen Teil ebenfalls Verankerungselemente, nämlich Beinchen mit Widerhaken 14, angeordnet. Die Beinchen sind im Wesentlichen parallel zum Anschlussteil 6 ausgerichtet. Durch die Beinchen mit den Widerhaken ist eine sehr gute und dauerhafte Positionierung der Vorrichtung 2 und damit ein optimaler Wundverschluss möglich.

Die Vorrichtungen der vorstehend beschriebenen Ausführungsbeispiele sind aus bioabbaubarem Material hergestellt. Dabei kommen insbesondere Lactidpolymere, aber auch andere im Körper leicht abbaubare Materialien in Frage. Um die Abbaurate der Lactidpolymere der voraussichtlichen Heilungszeit optimal anzupassen, kann dabei vorgesehen sein, die Lactidpolymere in einem bestimmten Mischungsverhältnis von D- zu L- Lactiden auszubilden.

### Bezugszeichenliste

- 2: Verschlussvorrichtung
- 4: scheibenförmiger Teil
- 6: Anschlussteil
- 8: Verankerungseinrichtung
- 10: Verankerungselemente
- 12: Widerhaken
- 14: ringförmiger Mittelteil
- 20: Trokar
- 22: Applizierelement/Applizierstab
- 30: Fascie/Muskel
- 32: Peritoneum
- 34: Fettgewebe

## Patentansprüche

1. Vorrichtung zum Verschliessen von Wunden, die durch einen Eingriff mittels eines Trokars (20) in Körpergewebe entstehen, insbesondere in der Bauchdecke oder im Rahmen von transluminalen endoskopischen Eingriffen durch natürliche Körperöffnungen, mit
■ einem scheibenförmigen Teil (4) zur innenseitigen Anlage an das Körpergewebe, insbesondere an das Peritoneum (30),
■ einem vorzugsweise stabförmigen Anschlussteil (6), der sich proximal an den scheibenförmigen Teil anschliesst und mit diesem verbindbar oder verbunden ist, und
■ einem Verbindungselement zum lösbaren Verbinden der Vorrichtung mit einem Applizierelement (22),
**dadurch gekennzeichnet, dass**
■ am proximalen Ende des Anschlussteils (6) eine Verankerungseinrichtung (8) angeordnet ist, mit der die Vorrichtung im Gewebe mittels Widerhaken (12) verankerbar ist.

2. Vorrichtung nach Anspruch 1, **dadurch gekennzeichnet, dass** die Verankerungseinrichtung (8) schirmartig ausgebildet ist und eine Vielzahl von Widerhaken (12), vorzugsweise drei bis fünf Widerhaken (12), aufweist.

3. Vorrichtung nach Anspruch 1 oder 2, **dadurch gekennzeichnet, dass** die Verankerungseinrichtung (8) aufklappbar ist.

4. Vorrichtung nach Anspruch 3, **dadurch gekennzeichnet, dass** die Verankerungseinrichtung (8) **dadurch** aufklappbar ist, dass die Widerhaken (12) jeweils um eine Achse senkrecht zum Anschlussteil (6) schwenkbar oder biegbar sind.

5. Vorrichtung nach einem der Ansprüche 1 bis 4, **dadurch gekennzeichnet, dass** der scheibenförmige Teil (4), der Anschlussteil (6) und die Verankerungseinrichtung (8) einstückig ausgebildet sind.

6. Vorrichtung nach einem der Ansprüche 1 bis 4, **dadurch gekennzeichnet, dass** die Vorrichtung zweiteilig aufgebaut ist.

7. Vorrichtung nach Anspruch 6, **dadurch gekennzeichnet, dass** der Anschlussteil (6), vorzugsweise an seinem proximalen Ende, im Wesentlichen stabförmig oder röhrenartig ausgebildet ist, und dass die Verankerungseinrichtung (8) einen ringförmigen, um den Anschlussteil (6) herum angeordneten Mittelteil (14) aufweist.

8. Vorrichtung nach Anspruch 7, **dadurch gekennzeichnet, dass** der Mittelteil längs des Anschlussteils (6) verschiebbar und vorzugsweise an dessen proximalen Ende arretierbar ist.

9. Vorrichtung nach Anspruch 8, **dadurch gekennzeichnet, dass** die Arretierbarkeit durch eine Wulst oder Auskragung am proximalen Ende des Anschlussteils (6) ausgebildet ist, über die der Mittelteil schiebbar und klemmend arretierbar ist.

10. Vorrichtung nach einem der Ansprüche 1 bis 9, **dadurch gekennzeichnet, dass** am scheibenförmigen Teil (4) Verankerungselemente (10) angeordnet sind, die im Wesentlichen parallel zum Anschlussteil (6) ausgerichtet sind und an ihrem Ende vorzugsweise Widerhaken aufweisen.

11. Vorrichtung nach einem der Ansprüche 1 bis 10, **dadurch gekennzeichnet, dass** der Anschlussteil (6) ein Gewinde aufweist, in oder auf welches ein Applizierelement (22) mit einem entsprechenden Gegengewinde schraubbar ist.

12. Vorrichtung nach einem der Ansprüche 1 bis 10, **dadurch gekennzeichnet, dass** der Anschlussteil (6) zumindest eine Ausnehmung aufweist, in die eine entsprechende, lösbare Einrichtung eines Applizierelements (22) eingreifen kann.

13. Vorrichtung nach einem der Ansprüche 1 bis 10, **dadurch gekennzeichnet, dass** der Anschlussteil (6) einen Schnellverschluss zum Verbinden und Lösen eines Applizierelements (22) aufweist.

14. Vorrichtung nach einem der Ansprüche 1 bis 13**, dadurch gekennzeichnet, dass** sie zumindest teilweise aus bioabbaubarem Material, vorzugsweise aus Lactidpolymeren, besteht.

15. Vorrichtung nach einem der vorstehenden Ansprüche 1 bis 14, **dadurch gekennzeichnet, dass** der Anschlussteil (6) röhrenartig ausgebildet ist, und an der Vorrichtung am proximalen und am distalen Ende Anschlusseinrichtungen zum Durchführen von Ver- und/oder Entsorgungselementen aufweist.
